(19) Europäisches Patentamt
European Patent Office
Office européen des brevets

(11) EP 1 239 815 B1

(12) EUROPÄISCHE PATENTSCHRIFT

(45) Veröffentlichungstag und Bekanntmachung des Hinweises auf die Patenterteilung:
**23.07.2008 Patentblatt 2008/30**

(21) Anmeldenummer: **00983340.1**

(22) Anmeldetag: **15.12.2000**

(51) Int Cl.:
***A61K 8/44*** *(2006.01)* ***A61K 8/64*** *(2006.01)*
***A61Q 5/06*** *(2006.01)*

(86) Internationale Anmeldenummer:
**PCT/EP2000/012805**

(87) Internationale Veröffentlichungsnummer:
**WO 2001/045653 (28.06.2001 Gazette 2001/26)**

(54) **FESTFÖRMIGES FÄRBEMITTEL FÜR KERATINFASERN**

SOLID COLORANT FOR KERATIN FIBRES

COLORANT SOLIDE POUR FIBRES DE KERATINE

(84) Benannte Vertragsstaaten:
**AT BE CH CY DE DK ES FI FR GB GR IE IT LI LU MC NL PT SE TR**

(30) Priorität: **20.12.1999 DE 19961910**

(43) Veröffentlichungstag der Anmeldung:
**18.09.2002 Patentblatt 2002/38**

(73) Patentinhaber: **Henkel AG & Co. KGaA**
**40589 Düsseldorf (DE)**

(72) Erfinder:
- **SCHULZE ZUR WIESCHE, Erik**
  **20251 Hamburg (DE)**
- **BOSSMANN, Britta**
  **40699 Erkrath (DE)**
- **HOLLENBERG, Detlef**
  **40699 Erkrath (DE)**
- **DREJA, Michael**
  **50931 Köln (DE)**

(56) Entgegenhaltungen:
**DE-B- 1 117 826** **GB-A- 524 293**

- **DATABASE WPI Week 197105 Derwent Publications Ltd., London, GB; AN 1971-09180s XP002161614 & JP 46 004280 B (TAISHO PHARM. CO. LTD.) in der Anmeldung erwähnt**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Formkörper zum Färben keratinischer Fasern, die neben Farbstoffvorprodukten und Oxidationsmittel zusätzlich ein Alkalisierungsmittel enthalten, die Verwendung dieser Mittel zur Herstellung von Haarfärbezubereitungen sowie ein Verfahren zum Färben keratinischer Fasern mit diesen Formkörpern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsflirbemittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B 1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Üblicherweise werden Haarfärbemittel in Form wäßriger Emulsionen oder Färbegele formuliert, die gegebenenfalls unmittelbar vor der Anwendung mit einer Oxidationsmittelzubereitung vermischt werden. Dieses Verfahren läßt aber hinsichtlich der Lagerstabilität der Formulierungen, der Dosierbarkeit und der einfachen Handhabung noch Wünsche offen.

In der JP-B-46-004280 wurde vorgeschlagen, Haarfärbemittel in Form einer Tablette zu konfektionieren, wobei die Färbezubereitung, enthaltend die Farbstoffe, und die Oxidationsmittelzubereitung sich nicht berühren. Es wurde vorgeschlagen, daß die Haarfärbekomponenten und die Oxidationsmittelzubereitung durch eine inerte Zwischenschicht, beispielsweise aus Avicel, Stärke oder Cellulosederivaten, getrennt werden. Üblicherweise sollen Tabletten stark verdichtet werden, um ein Auseinanderbrechen beziehungsweise starken Abrieb während der Herstellung, der Lagerung und des Transportes zu vermeiden. Allerdings bleibt das mit diesen Formkörpern erzielbare Färbeergebnis hinter der Färbeleistung herkömmlicher Zweikomponentensysteme zurück und diese Konfektionierungsform konnte sich bisher nicht auf dem Markt durchsetzen.

Es bestand daher die Aufgabe, die Färbeformkörper hinsichtlich ihrer färberischen Eigenschaften und ihres Lösungsverhaltens zu optimieren.

Es wurde nun überraschenderweise gefunden, daß durch Einsatz eines zusätzlichen Alkalisierungsmittels die erzielbaren Färbungen hinsichtlich ihrer Intensität und Echtheitseigenschaften deutlich verbessert werden können und sich die Formkörper durch eine deutlich verringerte Auflösungszeit auszeichnen.

Ein erster Gegenstand der vorliegenden Erfindung sind daher Formkörpern zur Färbung keratinischer Fasern, die in einem kosmetisch akzeptablen Träger mindestens ein Farbstoffvorprodukt, mindestens ein Oxidationsmittel und zusätzlich mindestens ein Alkalisierungsmittel enthalten.

Unter keratinischen Fasern sind erfindungsgemäß Pelze, Wolle, Federn und insbesondere menschliche Haare zu verstehen. Obwohl die erfindungsgemäßen Formkörper in erster Linie zum Färben von keratinischen Fasern geeignet sind, steht prinzipiell einer Verwendung auf anderen Gebieten nichts entgegen.

**Alkalisierungsmittel**

Als Alkalisierungsmittel werden Aminosäuren oder Oligopeptide mit mindestens einer Aminogruppe und einer Carboxy- oder eine Sulfogruppe eingesetzt, deren 2,5%ige wäßrige Lösung einen pH-Wert von größer als 9,0 aufweist.

In einer bevorzugten Ausführungsform der vorliegenden Erfindung kommen feste Alkalisierungsmittel zum Einsatz.

In einer weiteren Ausführungsform der vorliegenden Erfindung kann es bevorzugt sein, Alkalisierungsmittel einzusetzen, die sich durch eine gute Wasserlöslichkeit auszeichnen. Gut wasserlöslich sind erfindungsgemäß Verbindungen, von denen sich mindestens 5g in 100ml Wasser bei 15°C lösen. Besonders bevorzugt sind Verbindungen mit einer Wasserlöslichkeit von mehr als 7,5g in 100m1 Wasser bei 15°C.

Ferner haben sich die Alkalisierungsmittel als besonders bevorzugt erwiesen, die nach ihrer Einarbeitung in den erfindungsgemäßen Formkörpern nur einen geringen Partialdruck außerhalb des Formkörpers entwickeln.

Im Rahmen dieser Ausführungsform sind Aminocarbonsäuren besonders bevorzugt, insbesondere $\alpha$-Aminocarbonsäuren und $\omega$-Aminocarbonsäuren. Unter den $\alpha$-Aminocarbonsäuren sind wiederum Lysin und insbesondere Arginin besonders bevorzugt.

Die Aminosäuren können den erfindungsgemäßen Formkörpern bevorzugt in freier Form zugegeben werden. In einer Reihe von Fällen ist es jedoch auch möglich, die Aminosäuren in Salzform einzusetzen. Bevorzugte Salze sind dann die Verbindungen mit Halogenwasserstoffsäuren, insbesondere die Hydrochloride und die Hydrobromide.

Weiterhin können die Aminosäuren auch in Form von Oligopeptiden und Proteinhydrolysaten eingesetzt werden, wenn sichergestellt ist, daß die erforderlichen Mengen der erfindungsgemäß eingesetzten Aminosäuren darin enthalten sind. In diesem Zusammenhang wird auf die Offenbarung der DE-OS 22 15 303 verwiesen, auf die ausdrücklich Bezug genommen wird.

Ein ganz besonders bevorzugtes Alkalisierungsmittel ist Arginin, insbesondere in freier Form, aber auch als Hydrochlorid eingesetzt, da es neben seinen alkalischen Eigenschaften auch das Penetrationsvermögen der Farbstoffe deutlich erhöht.

Das Alkalisierungsmittel ist in den erfindungsgemäßen Formkörpern bevorzugt in Mengen von 0,5 bis 20 Gew.-%, insbesondere von 5 bis 15 Gew.-%, bezogen auf das gesamte Mittel, enthalten.

**Oxidationsmittel**

Obwohl die Wahl des Oxidationsmittels prinzipiell keinerlei Einschränkungen unterliegt kann es erfindungsgemäß bevorzugt sein, als Oxidationsmittel Anlagerungsprodukte von Wasserstoffperoxid, insbesondere an Harnstoff, Melamin oder Natriumborat, einzusetzen. Der Einsatz von Percarbamid ist besonders bevorzugt.

Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel.

So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Die Ausbildung der Färbung kann ferner dadurch unterstützt und gesteigert werden, daß dem Formkörper bestimmte Metallionen zugesetzt werden. Solche Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden. Es hat sich aber auch als praktikabel erwiesen, die Metallionen in Form ihrer Komplexe oder auch angelagert an Zeolithe zur Steigerung der Färbekraft zu verwenden.

**Farbstoffvorprodukte**

Hinsichtlich der in den erfindungsgemäßen Formkörpern eingesetzten Farbstoffvorprodukten unterliegt die vorliegende Erfindung keinerlei Einschränkungen. Die erfindungsgemäßen Formkörper können als Farbstoffvorprodukte

- Oxidationsfarbstoffvorprodukte vom Entwickler- und Kuppler-Typ, und
- Vorstufen naturanaloger Farbstoffe, wie Indol- und Indolin-Derivate,

sowie Mischungen von Vertretern dieser Gruppen enthalten.

Als Entwicklerkomponenten werden üblicherweise primäre aromatische Amine mit einer weiteren, in para- oder

ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1)

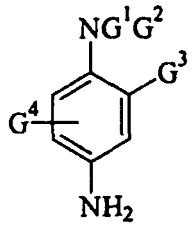

(E1)

wobei

- $G^1$ steht für ein Wasserstoffatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein ($C_1$- bis $C_4$)-Alkoxy-($C_1$- bis $C_4$)-alkylradikal, ein 4'-Aminophenylradikal oder ein $C_1$- bis $C_4$-Alkylradikal, das mit einer stickstoffhaltigen Gruppe, einem Phenyl- oder einem 4'-Aminophenylrest substituiert ist;
- $G^2$ steht für ein Wasserstoffatom, ein $C_1$- bis $C_4$-Alkylradikal, ein $C_1$- bis $C_4$-Menohydroxyalkylradikal, ein $C_2$- bis $C_4$-Polyhydroxyalkylradikal, ein ($C_1$- bis $C_4$)-Alkoxy-($C_1$- bis $C_4$)-alkylradikal oder ein $C_1$- bis $C_4$-Alkylradikal, das mit einer stickstoffhaltigen Gruppe substituiert ist;
- $G^3$ steht für ein Wasserstoffatom, ein Halogenatom, wie ein Chlor-, Brom, Jod- oder Fluoratom, ein $C_1$-bis $C_4$-Alkylradikal, ein $C_1$- bis $C_4$-Monohydroxyalkylradikal, ein $C_1$- bis $C_4$-Hydroxyalkoxyradikal, ein $C_1$- bis $C_4$-Acetylaminoalkoxyradikal, ein $C_1$- bis $C_4$- Mesylaminoalkoxyradikal oder ein $C_1$- bis $C_4$-Carbamoylaminoalkoxyradikal;
- $G^4$ steht für ein Wasserstoffatom, ein Halogenatom oder ein $C_1$- bis $C_4$-Alkylradikal oder
- wenn $G^3$ und $G^4$ in ortho-Stellung zueinander stehen, können sie gemeinsam eine verbrückende $\alpha,\omega$-Alkylendioxogruppe, wie beispielsweise einen Ethylendioxygruppe bilden.

Beispiele für die als Substituenten in den erfindungsgemäßen Verbindungen genannten, $C_1$- bis $C_4$-Alkylradikale sind die Gruppen Methyl, Ethyl, Propyl, Isopropyl und Butyl. Ethyl und Methyl sind bevorzugte Alkylradikale. Erfindungsgemäß bevorzugte $C_1$- bis $C_4$-Alkoxyradikale sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, $C_1$- bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N, N-Dipropyl-p-phenylendiamin, 4-Amino-3-methyl-(N,N-diethyl)-anilin, N,N-bis-(β-Hydroxyethyl)-p-phenylendiamin, 4-N, N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin. N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyeihyl)-p-phenylendiainin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiainin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

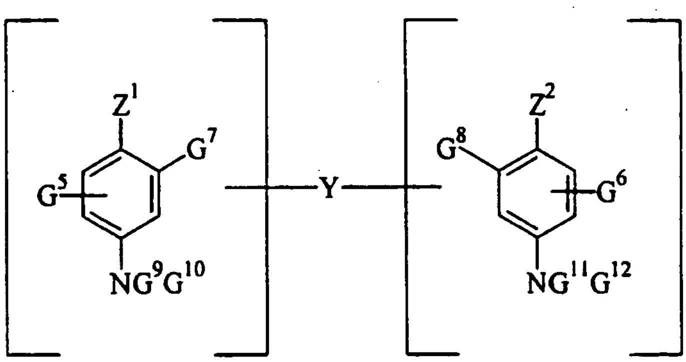

(E2)

wobei:

- $Z^1$ und $Z^2$ stehen unabhängig voneinander für ein Hydroxyl- oder $NH_2$-Radikal, das gegebenenfalls durch ein $C_1$- bis $C_4$-Alkylradikal, durch ein $C_1$- bis $C_4$-Hydroxyalkylradikal und/oder durch eine Verbrückung Y substituiert ist oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff, Schwefel- oder Stickstoffatomen unterbrochen oder beendet sein kann und eventuell durch ein oder mehrere Hydroxyl- oder $C_1$- bis $C_8$-Alkoxyradikale substituiert sein kann, oder eine direkte Bindung,
- $G^5$ und $G^6$ stehen unabhängig voneinander für ein Wasserstoff- oder Halogenatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein $C_1$- bis $C_4$-Aminoalkylradikal oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder ein $C_1$- bis $C_4$-Alkylradikal,

  mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-bis-(β-Hydroxyethyl)-N,N'-bis-(4'-aminophenyl)-1,3-diamino-propan-2-ol, N,N'-bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N-bis-(Ethyl)-N,N'-bis-(4'-amino-3'-methylphenyl)-ethylendiamin, Bis-(2-hydroxy-5-aminophenyl)-methan, 1,4-Bis-(4'-aminophenyl)-diaza-cycloheptan, N,N'-Bis-(2-hydroxy-5-aminobenryl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-bis-(β-Hydroxyethyl)-N,N'-bis-(4'-aminophenyl)-1,3-diamino-pmpan-2-ol, Bis-(2-hydroxy-5-aminophenyl)-methan, N,N'-Bis-(4-arninophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3)

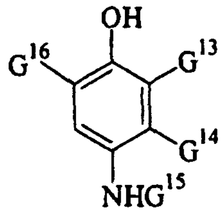

(E3)

wobei:

- $G^{13}$ steht für ein Wasserstoffatom, ein Halogenatom, ein $C_1$- bis $C_4$-Alkylradikal, ein $C_1$- bis $C_4$-Monohydroxyalkylradikal, ein ($C_1$- bis $C_4$)-Alkoxy-($C_1$- bis $C_4$)-alkylradikal, ein $C_1$- bis $C_4$-Aminoalkylradikal, ein Hydroxy-($C_1$- bis $C_4$)-alkylaminoradikal, ein $C_1$- bis $C_4$-Hydroxyalkoxyradikal, ein $C_1$- bis $C_4$-Hydroxyalkyl-($C_1$-bis $C_4$)-aminoalkylradikal oder ein (Di-$C_1$- bis $C_4$-Alkylamino)-($C_1$- bis $C_4$)-alkylradikal, und
- $G^{14}$ steht für ein Wasserstoff- oder Halogenatom, ein $C_1$- bis $C_4$-Alkylradikal, ein $C_1$- bis $C_4$-Monohydroxyalkylradikal, ein $C_2$- bis $C_4$-Polyhydroxyalkylradikal, ein ($C_1$- bis $C_4$)-Alkoxy-($C_1$- bis $C_4$)-alkylradikal, ein $C_1$- bis $C_4$-Aminoalkylradikal oder ein $C_1$- bis $C_4$-Cyanoalkylradikal,
- $G^{15}$ steht für Wasserstoff, ein $C_1$- bis $C_4$-Alkylradikal, ein $C_1$- bis $C_4$-Monohydroxyalkylradikal, ein $C_2$- bis $C_4$-Polyhydroxyalkylradikal, ein Phenylradikal oder ein Benzylradikal, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-l-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-l-methyl-4-methylaminopyrazol und 3,5-Diamino-4(β-hydroxyethyl) amino-1-methylpyrazol.

Bevorzugte Pyrazol-Pyrimidin-Derivate sind insbesondere die Derivate des Pyrazol-[1,5-a]-pyrimidin der folgenden Formel (V) und dessen tautomeren Formen, sofern ein tautomerisches Gleichgewicht besteht:

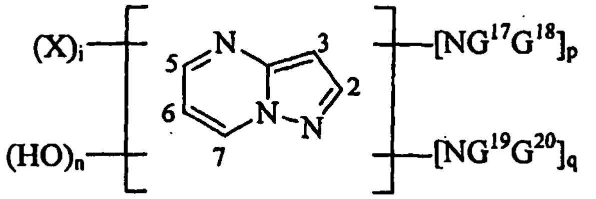

(E4)

wobei:

- $G^{17}$, $G^{18}$, $G^{19}$ und $G^{20}$ unabhängig voneinander stehen für ein Wasserstoffatom, ein $C_1$- bis $C_4$-Alkylradikal, ein Aryl-Radikal, ein $C_1$- bis $C_4$-Hydroxyalkylradikal, ein $C_2$- bis $C_4$-Polyhydroxyalkylradikal ein ($C_1$- bis $C_4$)-Alkoxy-($C_1$- bis $C_4$)-alkylradikal, ein $C_1$- bis $C_4$-Aminoalkylradikal, das gegebenenfalls durch ein Acetyl-Ureid- oder Sulfonyl-Radikal geschützt sein kann, ein ($C_1$- bis $C_4$)-Alkylamino-($C_1$- bis $C_4$)-alkylradikal, ein Di-[($C_1$- bis $C_4$)-alkyl]-($C_1$- bis $C_4$)-aminoalkylradikal, wobei die Dialkyl-Radikale gegebenenfalls einen Kohlenstoffzyklus oder einen Heterozyklus mit 5 oder 6 Kettengliedern bilden, ein $C_1$- bis $C_4$-Hydroxyalkyl- oder ein Di-($C_1$- bis $C_4$)-[Hydroxyalkyl]-($C_1$- bis $C_4$)-aminoalkylradikal,
- die X-Radikale stehen unabhängig voneinander für ein Wasserstoffatom, ein $C_1$- bis $C_4$-Alkylradikal, ein Aryl-Radikal, ein $C_1$- bis $C_4$-Hydroxyalkyladikal, ein $C_2$- bis $C_4$-Polyhydroxyalkylradikal, ein $C_1$- bis $C_4$-Aminoalkylradikal, ein ($C_1$- bis $C_4$)-Alkylamino-($C_1$- bis $C_4$)-alkylradikal, ein Di-[($C_1$- bis $C_4$)alkyll- ($C_1$- bis $C_4$)-aminoalkylradikal, wobei die Dialkyl-Radikale gegebenenfalls einen Kohlenstoffzyklus oder einen Heterozyklus mit 5 oder 6 Kettengliedern bilden, ein $C_1$- bis $C_4$-Hydroxyalkyl- oder ein Di-($C_1$- bis $C_4$-hydroxyalkyl)-aminoalkylradikal, ein Aminoradikal, ein $C_1$- bis $C_4$-Alkyl- oder Di-($C_1$- bis $C_4$-hydroxyalkyl)-aminoradikal, ein Halogenatom, eine Carboxylsäuregruppe oder eine Sulfonsäuregruppe,

  - i hat den Wert 0,1,2 oder 3,
  - p hat den Wert 0 oder 1,
  - q hat den Wert 0 oder 1 und
  - n hat den Wert 0 oder 1,
    mit der Maßgabe, daß
  - die Summe aus p + q ungleich 0 ist,
  - wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
  - wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyhmidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

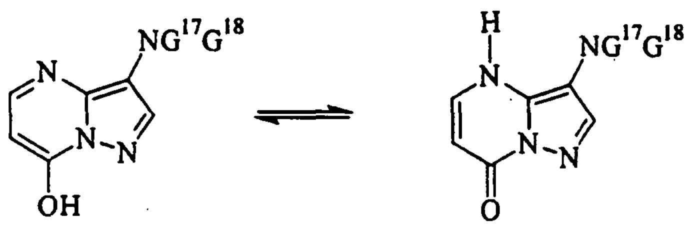

Unter den Pyrazol-[1,5-a]-pyrimidinen der obenstehenden Formel (E4) kann man insbesondere nennen:

- Pyrazol-[1,5-a]-pyrimidin-3,7-diamin;
- 2,5-Dimethyl pyrazol-[1,5-a]-pyrimidin-3,7-diamin;

- Pyrazol-[1,5-a]-pyrimidin-3,5-diamin;
- 2,7-Dimethyl pyrazol-[1,5-a]-pyrimidin-3,5-diamin;
- 3-Amino pyrazol-[1.5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;

sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Als Kupplerkomponenten werden in der Regel m-Phenylendiaminderivate, Naphthole. Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Anüno-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind:

- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)- 1 -methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-l-methylbenzot,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-l-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyriraidin, 4-Amino-2,6-dihydroxypyrimidin, 2,4-Diamino-6-hydroxypyrimidin, 2,4,6-Trihydroxypyrimidin, 2-Amino-4-methylpyrinüdin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Es ist nicht erforderlich, daß die Oxidationsfarbstoffvorprodukte oder die direktziehenden Farbstoffe jeweils

einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfarbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Armeimittel, Reformwaren und Körperpflegemittel e.V., Mannheim, Bezug genommen.

Die Oxidationsfarbstoffvorprodukte sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (Ia),

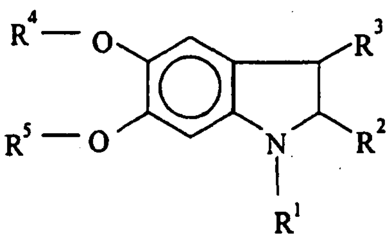

**(Ia)**

in der unabhängig voneinander
$R^1$ steht für Wasserstoff, eine $C_1$-$C_4$-Alkylgruppe oder eine $C_1$-$C_4$-Hydroxy-alkylgruppe,
$R^2$ steht für Wasserstoff oder eine -COOH-Gruppe, wobei die -COOH-Gruppe auch als Salz mit einem physiologisch verträglichen Kation vorliegen kann,
$R^3$ steht für Wasserstoff oder eine $C_1$-$C_4$-Alkylgruppe,
$R^4$ steht für Wasserstoff, eine $C_1$-$C_4$-Alkylgruppe oder eine Gruppe -CO-$R^6$, in der
$R^6$ steht für eine $C_1$-$C_4$-Alkylgruppe, und
$R^5$ steht für eine der unter $R^4$ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib),

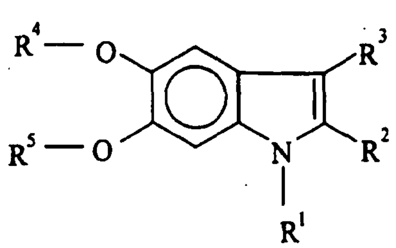

**(Ib)**

in der unabhängig voneinander
$R^1$ steht für Wasserstoff, eine $C_1$-$C_4$-Alkylgruppe oder eine $C_1$-$C_4$-Hydroxyalkylgruppe,

$R^2$ steht für Wasserstoff oder eine -COOH-Gruppe, wobei die -COOH-Gruppe auch als Salz mit einem physiologisch verträglichen Kation vorliegen kann,
$R^3$ steht für Wasserstoff oder eine $C_1$-$C_4$-Alkylgruppe,
$R^4$ steht für Wasserstoff, eine $C_1$-$C_4$-Alkylgruppe oder eine Gruppe -CO-$R^6$, in der
$R^6$ steht für eine $C_1$-$C_4$-Alkylgruppe, und
$R^5$ steht für eine der unter $R^4$ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Neben den Farbstoffvorprodukten können die erfindungsgemäßen Formkörper zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenal, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausfürungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

## Auflösungsbeschleuniger

Der erfindungsgemäße Formkörper enthält in einer bevorzugten Ausführungsform der vorliegenden Erfindung einen Auflösungsbeschleuniger. Der Begriff Auflösungsbeschleuniger umfaßt dabei Gas-entwickelnde Komponenten, vorgebildete und eingeschlossene Gase, Sprengmittel sowie deren Mischungen.

In einer Ausführungsform der vorliegenden Erfindung werden als Auflösungsbeschleuniger Gas-entwickelnde Komponenten eingesetzt. Diese Komponenten reagieren bei Kontakt mit Wasser miteinander unter in-situ Bildung von Gasen, die in der Tablette einen Druck erzeugen, der die Tablette in kleinere Partikel zerfallen läßt. Ein Beispiel für ein derartiges System sind spezielle Kombinationen von geeigneten Säuren mit Basen. Bevorzugt sind ein-, zwei- oder dreiwertige Säuren mit einem $pK_a$-Wert von 1,0 bis 6,9. Bevorzugte Säuren sind Citronensäure, Äpfelsäure, Maleinsäure, Malonsäure, Itaconsäure, Weinsäure, Oxalsäure, Glutarsäure, Glutaminsäure, Milchsäure, Fumarsäure, Glykolsäure sowie deren Mischungen. Besonders bevorzugt ist Citronensäure. Ganz besonders bevorzugt kann es sein, die Citronensäure in Teilchenform einzusetzen, wobei die Teilchen einen Durchmesser unterhalb von 1000μm, insbesondere kleiner als 700μm, ganz besonders bevorzugt kleiner als 400μm, aufweisen. Weitere alternative geeignete Säuren sind die Homopolymere oder Copolymere von Acrylsäure, Maleinsäure, Methacrylsäure oder Itaconsäure mit einem Molekulargewicht von 2000 bis 200 000. Besonders bevorzugt sind Homopolymere der Acrylsäure und Copolymere aus Acrylsäure und Maleinsäure. Bevorzugte Basen sind erfindungsgemäß Alkalimetallsilikate, Carbonate, Hydrogencarbonate sowie deren Mischungen. Metasilicate, Hydrogencarbonate und Carbonate sind besonders bevorzugt, Hydrogencarbonate sind ganz besonders bevorzugt. Besonders bevorzugt sind teilchenförmige Hydrogencarbonate mit einem Teilchendurchmesser von weniger als 1000μm, insbesondere weniger als 700μm, ganz besonders bevorzugt weniger als 400μm. Natrium oder Kaliumsalze der oben genannten Basen sind besonders bevorzugt. Diese Gas-entwickelnden Komponenten sind in den erfindungsgemäßen Färbeformkörpern bevorzugt in einer Menge von mindestens 10 Gew.-%, insbesondere von mindestens 20 Gew.-%, enthalten.

In einer weiteren Ausführungsform der vorliegenden Erfindung ist das Gas vorgebildet oder eingeschlossen,

so daß bei Einsetzen der Auflösung des Formkörpers die Gasentwicklung beginnt und die weitere Auflösung beschleunigt. Beispiele geeigneter Gase sind Luft, Kohlendioxid, $N_2O$, Sauerstoff und/oder weitere nicht-toxische, nichtbrennbare Gase.

In einer dritten, besonders bevorzugten Ausführungsform der vorliegenden Erfindung werden als Auflösungsbeschleuniger Desintegrationshilfsmittel, sogenannte Formkörpersprengmittel, in die Formkörper eingearbeitet, um die Zerfallszeiten zu verkürzen. Unter Formkörpersprengmitteln bzw. Zerfallsbeschleunigern werden gemäß Römpp (9. Auflage, Bd. 6, S. 4440) und Voigt "Lehrbuch der pharmazeutischen Technologie" (6. Auflage, 1987, S. 182-184) Hilfsstoffe verstanden, die für den raschen Zerfall von Formkörpern in Wasser oder Magensaft und für die Freisetzung der Pharmaka in resorbierbarer Form sorgen.

Diese Stoffe, die auch aufgrund ihrer Wirkung als "Spreng"mittel bezeichnet werden, vergrößern bei Wasserzutritt ihr Volumen (Quellung). Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden im Rahmen der vorliegenden Erfindung Desintegrationsmittel auf Cellulosebasis eingesetzt, so daß bevorzugte Formkörper ein solches Desintegrationsmittel auf Cellulosebasis in Mengen von 0,5 bis 50 Gew.-%, vorzugsweise 3 bis 30 Gew.-%, bezogen auf den gesamten Formkörper enthalten. Reine Cellulose weist die formale Bruttozusammensetzung $(C_6H_{10}O_5)_n$ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht als einzige Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Desintegrationshilfsmittel eingesetzte Cellulose wird vorzugsweise nicht in feinteiliger Form eingesetzt, sondern vor dem Zumischen zu den zu verpressenden Vorgemischen in eine gröbere Form überführt, beispielsweise granuliert oder kompaktiert. Die Teilchengrößen solcher Desintegrationsmittel liegen zumeist oberhalb 200 μm, vorzugsweise zu mindestens 90 Gew.% zwischen 300 und 1600 μm und insbesondere zu mindestens 90 Gew.% zwischen 400 und 1200 μm. Die erfindungsgemäßen Desintegrationshilfsmittel sind beispielsweise im Handel unter der Bezeichnung Arbocel® von der Firma Rettenmaier erhältlich. Ein bevorzugtes Desintegrationshilfsmittel ist beispielsweise Arbocel® TF-30-HG.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 μm aufweisen und beispielsweise zu Granulaten mit einer mittleren Teilchengröße von 200 μm kompaktierbar sind. Geeignete mikrokristalline Cellulose ist beispielsweise unter dem Handelsnamen Avicel® kommerziell erhältlich.

Die beschleunigte Auflösung der Formkörper kann erfindungsgemäß auch durch Vorgranulierung der weiteren Bestandteile des Formkörpers erreicht werden.

**Weitere Komponenten**

Neben den genannten Inhaltsstoffen können die erfindungsgemäßen Formkörper weiterhin alle für solche Zubereitungen bekannten Wirk-, Zusatz- und Hilfsstoffe enthalten. In vielen Fällen enthalten die Formkörper mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile

Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O-(Z)_X$. Diese Verbindungen sind beispielsweise unter dem Handelsnamen Plantacare® von Henkel erhältlich und sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden

Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen. Ein erfindungsgemäß besonders bevorzugtes Alkylglucosid ist das Handelsprodukt Plantacare® 1200G.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis (2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt

Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter. Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Formkörper bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten. Hinsichtlich der kationische Tenside sei auf die obigen Ausführungen verwiesen.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise

- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
  sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquatemium-2, Polyquatemium-10 und Polyquatemium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weiterhin enthalten die erfindungsgemäß verwendeten Zubereitungen bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe

definiert, deren Löslichkeit in Wasser bei 20 °C kleiner als 0,1 Gew.-% beträgt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 6 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäture, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Oxidation von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-caprylat erfindungsgemäß verwendbare Ölkomponenten dar, ebenso komplexe Ester wie z. B. das Diacetylglycerinmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaallkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die Ölkomponenten werden bevorzugt in Menden von 0,05 bis 10 Gew.-%, insbesondere von 0,1 bis 2 Gew.-% in den erfindungsgemäßen Formkörpern eingesetzt.

In einer bevorzugten Ausführungsform der vorliegenden Erfindung bildet sich bei Auflösung der Formkörper in Wasser ein Gel. Hierzu werden dem Formkörper Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, Silikate, wie sie beispielsweise unter den Handelsbezeichnungen Optigel® (Süd-Chemie) oder Laponite® (Solvay) vertrieben werden, oder vollsynthetische Hydrokolloide wie z.B. Polyvinylalkohol zugesetzt. Besonders bevorzugte Verdickungsmittel sind Xanthane, Alginate sowie hochsubstituierte Carboxymethylcellulosen.

Weitere Wirk-, Hilfs- und Zusatzstoffe sind beispielsweise

- zwitterionische und amphotere Polymere wie beispielsweise Acrylamidopropyl-trimethylammoniumchlorid/Acrylat-Copolymere und Octylacrylarnid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethylenglykol, Propylenglykol, Glycerin und Diethylenglykol,
- fasersmikturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwervvurzel,
- Cholesterin,
- Konsistenzgeber wie Zuckerester, Polyolester oder Polyolalkylether,
- Fette und Wachse wie Walrat, Bienenwachs, Montanwachs und Paraffine.
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für das Oxidationsmittel,
- Antioxidantien.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Es kann auch erfindungsgemäß bevorzugt sein, einzelne Wirkstoffe vor ihrer Einarbeitung in den Formkörper separat zu verkapseln; so ist es beispielsweise denkbar, besonders reaktive Komponenten oder auch die Duftstoffe in verkapselter Form einzusetzen.

**Formkörpergeometrien**

Die erfindungsgemäßen Formkörper können jedwede geometrische Form annehmen, wie beispielsweise konkave, konvexe, bikonkave, bikonvexe, kubische, tetragonale, orthorhombische, zylindrische, sphärische, zylindersegmentartige, scheibenförmige, tetrahedrale, dodecahedrale, octahedrale, konische, pyramidale, ellipsoide, fünf-, sieben- und achteckig-prismatische sowie rhomboedrische Formen. Auch völlig irreguläre Grundflächen wie Pfeil- oder Tierformen, Bäume, Wolken usw. können realisiert werden. Die Ausbildung als Tafel, die Stab- bzw. Barrenform, Würfel, Quader und entsprechende Raumelemente mit ebenen Seitenflächen sowie insbesondere zylinderförmige Ausgestaltungen mit kreisförmigem oder ovalem Querschnitt sind erfindungsgemäß bevorzugt. Diese zylinderförmige Ausgestaltung erfaßt dabei die Darbietungsform von der Tablette bis zu kompakten Zylinderstücken mit einem Verhältnis von Höhe zu Durchmesser größer 1. Weist der Basisformkörper Ecken und Kanten auf, so sind diese vorzugsweise abgerundet. Als zusätzliche optische Differenzierung ist eine Ausführungsform mit abgerundeten Ecken und abgeschrägten ("angefasten") Kanten bevorzugt.

In einer ersten bevorzugten Ausführungsform können die portionierten Preßlinge dabei jeweils als voneinander getrennte Einzelelemente ausgebildet sein, die der vorbestimmten Dosiermenge der Färbe- und/oder der Oxidationsmittel entspricht. Ebenso ist es aber möglich, Preßlinge auszubilden, die eine Mehrzahl solcher Masseneinheiten in einem Preßling verbinden, wobei insbesondere durch vorgegebene Sollbruchstellen die leichte Abtrennbarkeit portionierter kleinerer Einheiten vorgesehen ist. Die Ausbildung der portionierten Preßlinge als Tabletten in Zylinder- oder Quaderform kann zweckmäßig sein, wobei ein Durchmesser/Höhe-Verhältnis im Bereich von etwa 0,5 : 2 bis 2 : 0,5 bevorzugt ist. Handelsübliche Hydraulikpressen, Exzenterpressen oder Rundläuferpressen sind geeignete Vorrichtungen insbesondere zur Herstellung derartiger Preßlinge.

Die bevorzugte Raumform der erfindungsgemäßen Formkörper weist eine rechteckige Grundfläche auf, wobei die Höhe der Formkörper kleiner ist als die kleinere Rechteckseite der Grundfläche. Abgerundete Ecken sind bei dieser Angebotsform bevorzugt.

Ein weiterer bevorzugter Formkörper, der hergestellt werden kann, hat eine platten- oder tafelartige Struktur mit abwechselnd dicken langen und dünnen kurzen Segmenten, so daß einzelne Segmente von diesem "Riegel" an den Sollbruchstellen, die die kurzen dünnen Segmente darstellen, abgebrochen und derartig portioniert zum Einsatz kommen können. Dieses Prinzip des "riegelförmigen" Formkörpers kann auch in anderen geometrischen Formen, beispielsweise senkrecht stehenden Dreiecken, die lediglich an einer ihrer Seiten längsseits miteinander verbunden sind, verwirklicht werden.

In einer zweiten bevorzugten Ausführungsform werden die verschiedenen Komponenten nicht zu einer einheitlichen Tablette verpreßt, sondern bei der Tablettierung werden Formkörper erhalten, die mehrere Schichten, also mindestens zwei Schichten, aufweisen. Dabei ist es auch möglich, daß diese verschiedenen Schichten unterschiedliche Lösegeschwindigkeiten aufweisen. Hieraus können vorteilhafte anwendungstechnische Eigenschaften der Formkörper resultieren. Falls beispielsweise Komponenten in den Formkörpern enthalten sind, die sich wechselseitig negativ beeinflussen, so ist es möglich, die eine Komponente in der schneller löslichen Schicht zu integrieren und die andere Komponente in eine langsamer lösliche Schicht einzuarbeiten, so daß die Komponenten nicht bereits während des Lösevorgangs miteinander reagieren.

Es ist erfindungsgemäß besonders bevorzugt, wenn die Formkörper aus mindestens drei Schichten bestehen, wobei eine erste Schicht (A) die Farbstoffzubereitung und das Alkalisierungsmittel enthält, eine zweite Schicht (B) eine inerte Trennschicht darstellt und eine dritte Schicht (C) die Oxidationsmittelzubereitung enthält.

Der Schichtaufbau der Formkörper kann dabei sowohl stapelartig erfolgen, wobei ein Lösungsvorgang der inneren Schicht(en) an den Kanten des Formkörpers bereits dann erfolgt, wenn die äußeren Schichten noch nicht vollständig gelöst sind. Eine bevorzugte Stapelfolge ist (A), (B), (C).

Es kann aber auch gemäß einer weiteren Ausführungsform bevorzugt sein, wenn eine vollständige Umhüllung der inneren Schicht(en) durch die jeweils weiter außen liegende(n) Schicht(en) erreicht wird, was zu einer Verhinderung der frühzeitigen Lösung von Bestandteilen der inneren Schicht(en) führt. Bevorzugt sind Formkörper, bei denen die Schicht (A) vollständig von der Schicht (B) und diese wiederum vollständig von der Schicht (C) umhüllt ist. Ebenso können Formkörper bevorzugt sein, bei denen die Schicht (C) vollständig von der Schicht (B) und diese wiederum vollständig von der Schicht (A) umhüllt ist.

Ähnliche Effekte lassen sich auch durch Beschichtung ("coating") einzelner Bestandteile der zu verpressenden Zusammensetzung oder des gesamten Formkörpers erreichen. Hierzu können die zu beschichtenden Körper beispielsweise mit wäßrigen Lösungen oder Emulsionen bedüst werden, oder aber über das Verfahren der Schmelzbeschichtung einen Überzug erhalten.

Nach dem Verpressen weisen die Formkörper eine hohe Stabilität auf. Die Bruchfestigkeit zylinderförmiger Formkörper kann über die Meßgröße der diametralen Bruchbeanspruchung erfaßt werden. Diese ist bestimmbar nach

$$\sigma = \frac{2P}{\pi D t}$$

Hierin steht σ für die diametrale Bruchbeanspruchung (diametral fracture stress, DFS) in Pa, P ist die Kraft in N, die zu dem auf den Formkörper ausgeübten Druck führt, der den Bruch des Formkörpers verursacht, D ist der Formkörperdurchmesser in Meter und t ist die Höhe der Formkörper.

Die Formkörper der vorliegenden Erfindung weisen bevorzugterweise eine Dichte von 0,3g/cm$^3$ bis 2,0g/cm$^3$, insbesondere von 0,5g/cm$^3$ bis 1,1g/cm$^3$.

In einer dritten bevorzugten Ausführungsform bestehen die erfindungsgemäßen Formkörper aus einem, mit dem Begriff "Basisformkörper" beschriebenen, an sich durch bekannte Tablettiervorgänge hergestellten Formkörper,

der eine Mulde aufweist. Bevorzugterweise wird der Basisformkörper zuerst hergestellt und der weitere verpreßte Teil in einem weiteren Arbeitsschritt auf bzw. in diesen Basisformkörper auf- bzw. eingebracht. Das resultierende Produkt wird nachstehend mit dem Oberbegriff' "Muldenformkörper" oder "Muldentablette" bezeichnet.

Der Basisformkörper kann erfindungsgemäß prinzipiell alle realisierbaren Raumformen annehmen. Besonders bevorzugt sind die bereits oben genannten Raumformen. Die Form der Mulde kann frei gewählt werden, wobei erfindungsgemäß Formkörper bevorzugt sind, in denen mindestens eine Mulde eine konkave, konvexe, kubische, tetragonale, orthorhombische, zylindrische, sphärische, rylindersegmentartige, scheibenförmige, tetrahedrale, dodecahedrale, octahedrale, konische, pyramidale, ellipsoide, fünf-, sieben- und achteckig-prismatische sowie rhombohedrische Form annehmen kann. Auch völlig irreguläre Muldenformen wie Pfeil- oder Tierformen, Bäume, Wolken usw. können realisiert werden. Wie auch bei den Basisformkörpern sind Mulden mit abgerundeten Ecken und Kanten oder mit abgerundeten Ecken und angefasten Kanten bevorzugt.

Die Größe der Mulde im Vergleich zum gesamten Formkörper richtet sich nach dem gewünschten Verwendungszweck der Formkörper. Je nachdem, ob im zweiten verpreßten Teil eine geringere oder größere Menge an Aktivsubstanz enthalten sein soll, kann die Größe der Mulde variieren. Unabhängig vom Verwendungszweck sind Formkörper bevorzugt, bei denen das Gewichtsverhältnis von Basisformkörper zu Muldenfüllung im Bereich von 1:1 bis 100:1, vorzugsweise von 2:1 bis 80:1, besonders bevorzugt von 3:1 bis 50:1 und insbesondere von 4:1 bis 30:1 beträgt.

Ähnliche Aussagen lassen sich zu den Oberflächenanteilen machen, die der Basisformkörper bzw. die Muldenfüllung an der Gesamtoberfläche des Formkörpers ausmachen. Hier sind Formkörper bevorzugt, bei denen die Oberfläche der eingepreßten Muldenfüllung 1 bis 25 %, vorzugsweise 2 bis 20 %, besonders bevorzugt 3 bis 15 % und insbesondere 4 bis 10 % der Gesamtoberfläche des befüllten Basisformkörpers ausmacht.

Hat beispielsweise der Gesamtformkörper Abmessungen von 20 x 20 x 40 mm und somit eine Gesamtoberfläche von 40 $cm^2$, so sind Muldenfüllungen bevorzugt, die eine Oberfläche von 0,4 bis 10 $cm^2$, vorzugsweise 0,8 bis 8 $cm^2$, besonders bevorzugt von 1,2 bis 6 $cm^2$ und insbesondere von 1,6 bis 4 $cm^2$ aufweisen.

Die Muldenfüllung und der Basisformkörper sind vorzugsweise optisch unterscheidbar eingefärbt. Neben der optischen Differenzierung weisen Muldentabletten anwendungstechnische Vorteile einerseits durch unterschiedliche Löslichkeiten der verschiedenen Bereiche andererseits aber auch durch die getrennte Lagerung der Wirkstoffe in den verschiedenen Formkörperbereichen auf.

Formkörper, bei denen sich die eingepreßte Muldenfüllung langsamer löst als der Basisformkörper, sind erfindungsgemäß bevorzugt. Durch Inkorporation bestimmter Bestandteile kann einerseits die Löslichkeit der Muldenfüllung gezielt variiert werden, andererseits kann die Freisetzung bestimmter Inhaltsstoffe aus der Muldenfüllung zu Vorteilen im Färbeprozeß führen. Inhaltsstoffe, die bevorzugt zumindest anteilig in der Muldenfüllung lokalisiert sind, sind beispielsweise die weiter unten beschriebenen konditionierenden Wirkstoffe, Ölkörper, Vitamine und Pflanzenwirkstoffe.

**Tablettierung**

Die Herstellung der erfindungsgemäßen Formkörper erfolgt zunächst durch das trockene Vermischen der Bestandteile, die ganz oder teilweise vorgranuliert sein können, und anschließendes Informbringen, insbesondere Verpressen zu Tabletten, wobei auf bekannte Verfahren zurückgegriffen werden kann. Zur Herstellung der erfindungsgemäßen Formkörper wird das Vorgemisch in einer sogenannten Matrize zwischen zwei Stempeln zu einem festen Komprimat verdichtet. Dieser Vorgang, der im folgenden kurz als Tablettierung bezeichnet wird, gliedert sich in vier Abschnitte: Dosierung, Verdichtung (elastische Verformung), plastische Verformung und Ausstoßen.

Zunächst wird das Vorgemisch in die Matrize eingebracht, wobei die Füllmenge und damit das Gewicht und die Form des entstehenden Formkörpers durch die Stellung des unteren Stempels und die Form des Preßwerkzeugs bestimmt werden. Die gleichbleibende Dosierung auch bei hohen Fomikörperdurchsätzen wird vorzugsweise über eine volumetrische Dosierung des Vorgemischs erreicht. Im weiteren Verlauf der Tablettierung berührt der Oberstempel das Vorgemisch und senkt sich weiter in Richtung des Unterstempels ab. Bei dieser Verdichtung werden die Partikel des Vorgemisches näher aneinander gedrückt, wobei das Hohlraumvolumen innerhalb der Füllung zwischen den Stempeln kontinuierlich abnimmt. Ab einer bestimmten Position des Oberstempels (und damit ab einem bestimmten Druck auf das Vorgemisch) beginnt die plastische Verformung, bei der die Partikel zusammenfließen und es zur Ausbildung des Formkörpers kommt. Je nach den physikalischen Eigenschaften des Vorgemisches wird auch ein Teil der Vorgemischpartikel zerdrückt, und es kommt bei noch höheren Drücken zu einer Sinterung des Vorgemischs. Bei steigender Preßgeschwindigkeit, also hohen Durchsatzmengen, wird die Phase der elastischen Verformung immer weiter verkürzt, so daß die entstehenden Formkörper mehr oder minder große Hohlräume aufweisen können. Im letzten Schritt der Tablettierung wird der fertige Formkörper durch den Unterstempel aus der Matrize herausgedrückt und durch nachfolgende Transporteinrichtungen wegbefördert. Zu diesem Zeitpunkt ist lediglich das Gewicht des Formkörpers endgültig festgelegt, da die Preßlinge aufgrund physikalischer Prozesse (Rückdehnung, kristallographische Effekte, Abkühlung etc.) ihre Form und Größe noch ändern können.

Die Tablettierung erfolgt in handelsüblichen Tablettenpressen, die prinzipiell mit Einfach- oder Zweifachstempeln ausgerüstet sein können. Im letzteren Fall wird nicht nur der Oberstempel zum Druckaufbau verwendet, auch der Unterstempel bewegt sich während des Preßvorgangs auf den Oberstempel zu, während der Oberstempel nach unten drückt. Für kleine Produktionsmengen werden vorzugsweise Exzentertablettenpressen verwendet, bei denen der oder die Stempel an einer Exzenterscheibe befestigt sind, die ihrerseits an einer Achse mit einer bestimmten Umlaufgeschwindigkeit montiert ist. Die Bewegung dieser Preßstempel ist mit der Arbeitsweise eines üblichen Viertaktmotors vergleichbar. Die Verpressung kann mit je einem Ober- und Unterstempel erfolgen, es können aber auch mehrere Stempel an einer Exzenterscheibe befestigt sein, wobei die Anzahl der Matrizenbohrungen entsprechend erweitert ist. Die Durchsätze von Exzenterpressen variieren ja nach Typ von einigen hundert bis maximal 3000 Tabletten pro Stunde.

Für größere Durchsätze wählt man Rundlauftablettenpressen, bei denen auf einem sogenannten Matrizentisch eine größere Anzahl von Matrizen kreisförmig angeordnet ist. Die Zahl der Matrizen variiert je nach Modell zwischen 6 und 55, wobei auch größere Matrizen im Handel erhältlich sind. Jeder Matrize auf dem Matrizentisch ist ein Ober- und Unterstempel zugeordnet, wobei wiederum der Preßdruck aktiv nur durch den Ober- bzw. Unterstempel, aber auch durch beide Stempel aufgebaut werden kann. Der Matrizentisch und die Stempel bewegen sich um eine gemeinsame senkrecht stehende Achse, wobei die Stempel mit Hilfe schienenartiger Kurvenbahnen während des Umlaufs in die Positionen für Befüllung, Verdichtung, plastische Verformung und Ausstoß gebracht werden. An den Stellen, an denen eine besonders gravierende Anhebung bzw. Absenkung der Stempel erforderlich ist (Befüllen, Verdichten, Ausstoßen), werden diese Kurvenbahnen durch zusätzliche Niederdruckstücke, Niederzugschienen und Aushebebahnen unterstützt. Die Befüllung der Matrize erfolgt über eine starr angeordnete Zufuhreinrichtung, den sogenannten Füllschuh, der mit einem Vorratsbehälter für das Vorgemisch verbunden ist. Der Preßdruck auf das Vorgemisch ist über die Preßwege für Ober- und Unterstempel individuell einstellbar, wobei der Druckaufbau durch das Vorbeirollen der Stempelschaftköpfe an verstellbaren Druckrollen geschieht.

Rundlaufpressen können zur Erhöhung des Durchsatzes auch mit zwei Füllschuhen versehen werden, wobei zur Herstellung einer Tablette nur noch ein Halbkreis durchlaufen werden muß. Zur Herstellung zwei- und mehrschichtiger Formkörper werden mehrere Füllschuhe hintereinander angeordnet, ohne daß die leicht angepreßte erste Schicht vor der weiteren Befüllung ausgestoßen wird. Durch geeignete Prozeßführung sind auf diese Weise auch Mantel- und Punkttabletten herstellbar, die einen zwiebelschalenartigen Aufbau haben, wobei im Falle der Punkttabletten die Oberseite des Kerns bzw. der Kernschichten nicht überdeckt wird und somit sichtbar bleibt. Auch Rundlauftablettenpressen sind mit Einfach- oder Mehrfachwerkzeugen ausrüstbar, so daß beispielsweise ein äußerer Kreis mit 50 und ein innerer Kreis mit 35 Bohrungen gleichzeitig zum Verpressen benutzt werden. Die Durchsätze moderner Rundlauftablettenpressen betragen über eine Million Formkörper pro Stunde.

Bei der Tablettierung mit Rundläuferpressen hat es sich als vorteilhaft erwiesen, die Tablettierung mit möglichst geringen Gewichtschwankungen der Tablette durchzuführen. Auf diese Weise lassen sich auch die Härteschwankungen der Tablette reduzieren. Geringe Gewichtschwankungen können auf folgende Weise erzielt werden:

- Verwendung von Kunststoffeinlagen mit geringen Dickentoleranzen
- Geringe Umdrehungszahl des Rotors
- Große Füllschuhe
- Abstimmung des Füllschuhflügeldrehzahl auf die Drehzahl des Rotors
- Füllschuh mit konstanter Pulverhöhe
- Entkopplung von Füllschuh und Pulvervorlage

Zur Verminderung von Stempelanbackungen bieten sich sämtliche aus der Technik bekannte Antihaftbeschichtungen an. Besonders vorteilhaft sind Kunststoffbeschichtungen, Kunststoffeinlagen oder Kunststoffstempel. Auch drehende Stempel haben sich als vorteilhaft erwiesen, wobei nach Möglichkeit Ober- und Unterstempel drehbar ausgeführt sein sollten. Bei drehenden Stempeln kann auf eine Kunststoffeinlage in der Regel verzichtet werden. Hier sollten die Stempeloberflächen elektropoliert sein.

Es zeigte sich weiterhin, daß lange Preßzeiten vorteilhaft sind. Diese können mit Druckschienen, mehreren Druckrollen oder geringen Rotordrehzahlen eingestellt werden. Da die Härteschwankungen der Tablette durch die Schwankungen der Preßkräfte verursacht werden, sollten Systeme angewendet werden, die die Preßkraft begrenzen. Hier können elastische Stempel, pneumatische Kompensatoren oder federnde Elemente im Kraftweg eingesetzt werden. Auch kann die Druckrolle federnd ausgeführt werden.

Im Rahmen der vorliegenden Erfindung geeignete Tablettiermaschinen sind beispielsweise erhältlich bei den Firmen Apparatebau Holzwarth GbR, Asperg, Wilhelm Fette GmbH, Schwarzenbek, Fann Instruments Company, Houston, Texas (USA), Hofer GmbH, Weil, Horn & Noack Pharmatechnik GmbH, Worms, IMA Verpackungssysteme GmbH Viersen, KILIAN, Köln, KOMAGE, Kell am See, KORSCH Pressen AG, Berlin, sowie Romaco GmbH, Worms. Weitere Anbieter sind beispielsweise Dr. Herbert Pete, Wien (AT), Mapag Maschinenbau AG, Bern (CH), BWI Manesty, Liverpool (GB), I. Holand Ltd., Nottingham (GB), Courtoy N.V., Halle (BE/LU) sowie Mediopharm Kamnik (SI). Besonders geeignet ist beispielsweise die Hydraulische Doppeldruckpresse HPF 630 der Firma LAEIS, D. Tablettierwerkzeuge sind beispielsweise von den Firmen Adams Tablettierwerkzeuge, Dresden, Wilhelm Fett GmbH, Schwarzenbek, Klaus Hammer, Solingen, Herber % Söhne GmbH, Hamburg, Hofer GmbH, Weil, Horn & Noack, Pharmatechnik GmbH, Worms, Ritter Pharamatechnik GmbH, Hamburg, Romaco, GmbH, Worms und Notter Werkzeugbau, Tamm erhältlich. Weitere Anbieter sind z.B. die Senss AG, Reinach (CH) und die Medicopharm, Kamnik (SI).

Das Verfahren zur Herstellung der Formkörper ist aber nicht darauf beschränkt, daß lediglich ein teilchenförmiges Vorgemisch zu einem Formkörper verpreßt wird. Vielmehr läßt sich das Verfahren auch dahingehend erweitern, daß man in an sich bekannter Weise mehrschichtige Formkörper herstellt, indem man zwei oder mehrere Vorgemische bereitet, die aufeinander verpreßt werden. Hierbei wird das zuerst eingefüllte Vorgemisch leicht vorverpreßt, um eine glatte und parallel zum Formkörperboden verlaufende Oberseite zu bekommen, und nach Einfüllen des zweiten Vorgemischs zum fertigen Formkörper endverpreßt. Bei drei- oder mehrschichtigen Formkörpern erfolgt nach jeder Vorgemisch-Zugabe eine weitere Vorverpressung, bevor nach Zugabe des letzten Vorgemischs der Formkörper endverpreßt wird.

Die Verpressung der teilchenförmigen Zusammensetzung in die Mulde kann analog zur Herstellung der Basisformkörper auf Tablettenpressen erfolgen. Bevorzugt ist eine Verfahrensweise, bei der erst die Basisformkörper mit Mulde hergestellt, dann befüllt und anschließend erneut verpreßt werden. Dies kann durch Ausstoß der Basisformkörper aus einer ersten Tablettenpresse, Befüllen und Transport in eine zweite Tablettenpresse geschehen, in der die Endverpressung erfolgt. Alternativ kann die Endverpressung auch durch Druckrollen, die über die auf einem Transportband befindlichen Formkörper rollen, erfolgen. Es ist aber auch möglich, eine Rundläufertablettenpresse mit unterschiedlichen Stempelsätzen zu versehen, so das ein erster Stempelsatz Vertiefungen in die Formkörper einpreßt und der zweite Stempelsatz nach Befüllung durch Nachverpressung für eine plane Formkörperoberfläche sorgt.

**Verpackung**

Die erfindungsgemäß hergestellten Formkörper können - wie oben beschrieben - ganz oder teilweise mit einer Beschichtung versehen werden. Verfahren, in denen eine Nachbehandlung im Aufbringen einer Coatingschicht auf die Formkörperfläche(n), in der/denen sich die befüllte(n) Mulde(n) befinden, oder im Aufbringen einer Coatingschicht auf den gesamten Formkörper besteht, sind erfindungsgemäß bevorzugt.

Die erfindungsgemäßen Formkörper können nach der Herstellung verpackt werden, wobei sich der Einsatz bestimmter Verpackungssysteme besonders bewährt hat, da diese Verpackungssysteme einerseits die Lagerstabilität der Inhaltsstoffe erhöhen, andererseits gegebenenfalls aber auch die Langzeithaftung der Muldenfüllung deutlich verbessern. Der Begriff "Verpackungssystem" kennzeichnet dabei im Rahmen der vorliegenden Erfindung immer die Primärverpackung der Formkörper, d.h. die Verpackung, die an ihrer Innenseite direkt mit der Formkörperoberfläche in Kontakt ist. An eine optionale Sekundärverpackung werden keinerlei Anforderungen gestellt, so daß hier alle üblichen Materialien und Systeme eingesetzt werden können.

Erfindungsgemäß bevorzugt sind Verpackungssysteme, die nur eine geringe Feuchtigkeitsdurchlässigkeit aufweisen. Auf diese Weise läßt sich das Färbevermögen der erfindungsgemäßen Formkörper über einen längeren Zeitraum erhalten, auch wenn beispielsweise hygroskopische Komponenten in den Formkörpern eingesetzt werden. Besonders bevorzugt sind Verpackungssysteme, die eine Feuchtigkeitsdampfdurchlässigkeitsrate von 0,1 $g/m^2$/Tag bis weniger als 20 $g/m^2$/Tag aufweist, wenn das Verpackungssystem bei 23°C und einer relativen Gleichgewichtsfeuchtigkeit von 85% gelagert wird. Die genannten Temperatur- und Feuchtigkeitsbedingungen sind die Prüfbedingungen, die in der DIN-Norm 53122 genannt werden, wobei laut DIN 53122 minimale Abweichungen zulässig sind (23 ± 1°C, 85 ± 2% rel. Feuchte). Die Feuchtigkeitsdampfdurchlässigkeitsrate eines gegebenen Verpackungssystems bzw. Materials läßt sich nach weiteren Standardmethoden bestimmen und ist beispielsweise auch im ASTM-Standard E-96-53T ("Test for measuring Water Vapor transmission of Materials in Sheet form") und im TAPPI Standard T464 m-45 ("Water Vapor Permeability of Sheet Materials at high temperature an Humidity") beschrieben. Das Meßprinzip gängiger Verfahren beruht dabei auf der Wasseraufnahme von wasserfreiem Calciumchlorid, welches in einem Behälter in der entsprechenden Atmosphäre gelagert wird, wobei der Behälter an der Oberseite mit dem zu testenden Material verschlossen ist. Aus der Oberfläche des Behälters, die mit dem zu testenden Material verschlossen ist (Permeationsfläche), der Gewichtszunahme des Calciumchlorids und der Expositionszeit läßt sich die Feuchtigkeitsdampfdurchlässigkeitsrate nach

$$FDDR = \frac{24 \cdot 10000}{A} \cdot \frac{x}{y} \left[ g / m^2 / 24h \right]$$

berechnen, wobei A die Fläche des zu testenden Materials in $cm^2$, x die Gewichtszunahme des Calciumchlorids in g und y die Expositionszeit in h bedeutet.

Die relative Gleichgewichtsfeuchtigkeit, oft als "relative Luftfeuchtigkeit" bezeichnet, beträgt bei der Messung der Feuchtigkeitsdampfdurchlässigkeitsrate im Rahmen der vorliegenden Erfindung 85% bei 23°C. Die Aufnahmefähigkeit von Luft für Wasserdampf steigt mit der Temperatur bis zu einem jeweiligen Höchstgehalt, dem sogenannten Sättigungsgehalt, an und wird in g/$m^3$ angegeben. So ist beispielsweise 1 $m^3$ Luft von 17° mit 14,4 g Wasserdampf gesättigt, bei einer Temperatur von 11° liegt eine Sättigung schon mit 10 g Wasserdampf vor. Die relative Luftfeuchtigkeit ist das in Prozent ausgedrückte Verhältnis des tatsächlich vorhandenen Wasserdampf-Gehalts zu dem der herrschenden Temperatur entsprechenden Sättigungs-Gehalt. Enthält beispielsweise Luft von 17° 12 g/$m^3$ Wasserdampf, dann ist die relative Luftfeuchtigkeit = (12/14,4)·100 = 83%. Kühlt man diese Luft ab, dann wird die Sättigung (100% r. L.) beim sogenannten Taupunkt (im Beispiel: 14°) erreicht, d.h., bei weiterem Abkühlen bildet sich ein Niederschlag in Form von Nebel (Tau). Zur quantitativen Bestimmung der Feuchtigkeit benutzt man Hygrometer und Psychrometer.

Die relative Gleichgewichtsfeuchtigkeit von 85% bei 23°C läßt sich beispielsweise in Laborkammem mit Feuchtigkeitskontrolle je nach Gerätetyp auf +/- 2% r.L. genau einstellen. Auch über gesättigten Lösungen bestimmter Salze bilden sich in geschlossenen Systemen bei gegebener Temperatur konstante und wohldefinierte relative Luftfeuchtigkeiten aus, die auf dem Phasen-Gleichgewicht zwischen Partialdruck des Wassers, gesättigter Lösung und Bodenkörper beruhen.

Die Kombinationen aus Formkörper und Verpackungssystem können selbstverständlich ihrerseits in Sekundärverpackungen, beispielsweise Kartonagen oder Trays, verpackt werden, wobei an die Sekundärverpackung keine weiteren Anforderungen gestellt werden müssen. Die Sekundärverpackung ist demnach möglich, aber nicht notwendig.

Das Verpackungssystem umschließt je nach Ausführungsform der Erfindung einen oder mehrere Formkörper. Es ist dabei erfindungsgemäß bevorzugt, entweder einen Formkörper derart zu gestalten, daß er eine Anwendungseinheit des Färbemittels umfaßt, und diesen Formkörper einzeln zu verpacken, oder die Zahl an Formkörpern in eine Verpackungseinheit einzupacken, die in Summe eine Anwendungseinheit umfaßt. Dieses Prinzip läßt sich selbstverständlich erweitern, so daß erfindungsgemäß Kombinationen auch drei, vier, fünf oder noch mehr Formkörper in einer Verpackungseinheit enthalten können. Selbstverständlich können zwei oder mehr Formkörper in einer Verpackung unterschiedliche Zusammensetzungen aufweisen. Auf diese Weise ist es möglich, bestimmte Komponenten räumlich voneinander zu trennen, um beispielsweise Stabilitätsprobleme zu vermeiden.

Das Verpackungssystem der erfindungsgemäßen Kombination kann aus den unterschiedlichsten Materialien bestehen und beliebige äußere Formen annehmen. Aus ökonomischen Gründen und aus Gründen der leichteren Verarbeitbarkeit sind allerdings Verpackungssysteme bevorzugt, bei denen das Verpackungsmaterial ein geringes Gewicht hat, leicht zu verarbeiten und kostengünstig sowie ökologisch verträglich ist.

In einer ersten erfindungsgemäß bevorzugten Kombinationen besteht das Verpackungssystem aus einem Sack oder Beutel aus einschichtigem oder laminiertem Papier und/oder Kunststoffolie. Dabei können die Formkörper unsortiert, d.h. als lose Schüttung, in einen Beutel aus den genannten Materialien gefüllt werden. Es ist aber aus ästhetischen Gründen und zur Sortierung der Kombinationen in Sekundärverpackungen bevorzugt, die Formkörper einzeln oder zu mehreren sortiert in Säcke oder Beutel zu füllen. Diese Verpackungssystme können dann - wiederum vorzugsweise sortiert - optional in Umverpackungen verpackt werden, was die kompakte Angebotsform des Formkörpers unterstreicht.

Die bevorzugt als Verpackungssystem einzusetzenden Säcke bzw. Beutel aus einschichtigem oder laminiertem Papier bzw. Kunststoffolie können auf die unterschiedlichste Art und Weise gestaltet werden, beispielsweise als aufgeblähte Beutel ohne Mittelnaht oder als Beutel mit Mittelnaht, welche durch Hitze (Heißverschmelzen), Klebstoffe oder Klebebänder verschlossen werden. Einschichtige Beutel- bzw. Sackmaterialien sind die bekannten Papiere, die gegebenenfalls imprägniert sein können, sowie Kunststoffolien, welche gegebenenfalls coextrudiert sein können. Kunststoffolien, die im Rahmen der vorliegenden Erfindung als Verpackungssystem eingesetzt werden können, sind beispielsweise in Hans Domininghaus "Die Kunststoffe und ihre Eigenschaften", 3. Auflage, VDI Verlag, Düsseldorf, 1988, Seite 193, angegeben. Die dort gezeigte Abbildung 111 gibt gleichzeitig Anhaltspunkte zur Wasserdampfdurchlässigkeit der genannten Materialien.

Obwohl es möglich ist, neben den genannten Folien bzw. Papieren auch wachsbeschichtete Papiere in Form von Kartonagen als Verpackungssystem für die Formkörper einzusetzen, ist es im Rahmen der vorliegenden Erfindung bevorzugt, wenn das Verpackungssystem keine Kartons aus wachsbeschichtetem Papier umfaßt.

An die optionale Sekundärverpackung werden keinerlei Anforderungen gestellt, so daß hier alle üblichen Materialien und Systeme eingesetzt werden können.

Ebenfalls bevorzugt sind Ausführungsformen, bei denen das Verpackungssystem wiederverschließbar ausgeführt ist. Es hat sich beispielsweise als praktikabel erwiesen, als Verpackungssystem ein wiederverschließbares Röhrchen aus Glas, Kunststoff oder auch Metall zu verwenden. Auf diese Weise ist es möglich, die Dosierbarkeit der Haarfärbeprodukte zu optimieren, so daß der Verbraucher beispielsweise angeleitet werden kann, pro definierter Haarlängeneinheit jeweils einen Formkörper zu verwenden. Auch Verpackungssysteme, die eine Microperforation aufweisen, lassen sich erfindungsgemäß mit Vorzug realisieren.

Ein zweiter Gegenstand der vorliegenden Erfindung ist die Verwendung der oben beschriebenen Formkörper

zur Herstellung eines Mittels zur Färbung keratinischer Fasern.

Ein dritter Gegenstand der vorliegenden Erfindung ist ein Verfahren zum Färben keratinischer Fasern, bei dem ein oder mehrere Formkörper in Wasser gelöst werden, die resultierende Zubereitung auf die Fasern aufgetragen und nach einer Einwirkzeit wieder abgespült wird.

Obwohl es prinzipiell bevorzugt ist, alle für die Haarfärbung benötigten Wirkstoffe bis auf das Lösemittel in die Formkörper einzuarbeiten, kann es erfindungsgemäß dennoch bevorzugt sein, der durch Lösung der Tablette in Wasser erhaltenen Zubereitung weitere Wirkstoffe zuzusetzen. Beispielsweise kann der Verbraucher angeleitet werden, zur weiteren Nuancierung eine spezielle Färbekomponente oder zur weiteren Aufhellung eine weitere Oxidationskomponente zuzusetzen. Es kann auch erfindungsgemäß bevorzugt sein, dieser Zubereitung unmittelbar vor der Anwendung weitere in dem Formkörper nicht stabil konfektionierbare Wirkstoffe, wie beispielsweise spezielle Enzymzubereitungen oder flüssige Pflegekomponenten, zuzusetzen.

Dabei können die Anwendungstemperaturen in einem Bereich zwischen 15 und 40 °C, bevorzugt bei der Temperatur der Kopfhaut, liegen. Die Einwirkungszeit beträgt üblicherweise ca. 5 bis 45, insbesondere 15 bis 30, Minuten. Sofern kein stark tensidhaltiger Träger verwendet wurde, kann es bevorzugt sein, die derart behandelten Haare anschließend mit einem Shampoo zu reinigen.

## Beispiele

Alle Mengenangaben in den Beispielen sind in Gewichtsprozent.

Es wurden die folgenden Haarfarbefonnkörper hergestellt:

### Beispiel 1

| Phase 1 | |
|---|---|
| 3-Methyl-4-aminophenol | 0,1 |
| 2-Amino-3-hydroxypyridin | 0,1 |
| Texapon® K 1296 PLV[1] | 2,0 |
| Avicel® pH 102[2] | 30,0 |
| Ammoniumsulfat | 1,0 |
| Arginin | 10,0 |
| D(+)-Lactose | ad 100 |
| Optigel® SH[3] | 7,5 |
| Amaze® [4] | 2,5 |
| Jaguar® HP 120[5] | 7,5 |
| Luviskol® K30[6] | 2,5 |
| **Phase 2** | |
| D(+)-Lactose | 100 |

[1] Laurylsulfat, Natriumsalz (Pulver; INCI-Bezeichnung: Sodium Lauryl Sulfate) (HENKEL)

[2] mikrokristalline Cellulose (FMC Corporation)

[3] synthetisches Magnesiumschichtsilikat (Süd Chemie)

[4] modifizierte Stärke (INCI-Bezeichnung: Com Starch modified) (National Starch)

[5] Hydroxypropylguar (INCI-Bezeichnung: Hydroxypropyl Guar) (Rhodia)

[6] Polyvinylpyrrolidon (INCI-Hezeichnung: PVP) (BASF)

| Phase 3 | |
|---|---|
| Carbamid-Perhydrat[7] | 100 |

[7] Harnstoff-Wasserstoffperoxid (INCI-Bezeichnung: Urea Peroxide) (Peroxid Chemie)

1,8g der Zubereitung der Phase I wurden in einen Preßzylinder einer Tablettiermaschine der Firma Fann Instrument Company eingeführt und mit einem Stempel leicht angedrückt. Danach wurden 0,2g der Phase 2 über die Phase 1 geschichtet und ebenfalls mit einem Stempel leicht angedrückt. Anschließend wurden 2,0g der Phase 3 über den Preßling geschichtet und ebenfalls leicht angedrückt.

Abschließend wurde die gesamte Tablette mit einem Preßdruck von 13,3KN für 30sec gepreßt, bevor sie aus der Presse entnommen wurde. Dies entspricht bei einem Durchmesser der Tablette von 2,9cm einem Druck von 2013N/cm$^2$ Tablettenoberfläche. Die Tablette wies eine Dichte von 0,86g/cm$^3$ auf.

Ausfärbung:

Die derart hergestellte Tablette wurde unter Rühren (mit einem Glasstab) bei Raumtemperatur in 20 ml Wasser innerhalb von 2 Minuten unter Bildung eines Färbegels gelöst. Ohne Rühren wurden 4 Minuten bis zur vollständigen Lösung der Tablette benötig. Das so erhaltene Gel wurde auf 5cm lange Strähnen standardisierten, zu 80% ergrauten, aber nicht besonders vorbehandelten Menschenhaares (Kerling) aufgetragen. Nach 30min Einwirkzeit bei 32°C wurde das Haar gespült, mit einem üblichen Haarwaschmittel ausgewaschen und anschließend getrocknet. Die Fasern wiesen eine mittelblonde Färbung auf.

## Beispiel 2

| Phase 1 | |
|---|---|
| p-Toluylendiamin-sulfat | 3,2 |
| 4-Chlorresorcin | 1,4 |
| Resorcin | 0,8 |
| 4-Amino-3-methylphenol | 0,6 |
| 5-Amino-2-methylphenol | 0,5 |
| Texapon® K 1296 PLV | 2,0 |
| Avicel® pH 102 | 30,0 |
| Ammoniumsulfat | 1,0 |
| Arginin | 10,0 |
| D(+)-Lactose | ad 100 |
| Optigel® SH | 15,0 |
| Amaze® | 5,0 |
| Jaguar® HP 120 | 15,0 |
| Luviskol® K30 | 5,0 |

Analog zur Herstellvorschrift des Beispiel 1 wurde eine Tablette gepreßt und mit dem resultierende Färbegel entsprechende Strähnen gefärbt. Die Strähnen wiesen eine haselnussbraune Färbung auf.

## Beispiel 3

| Phase 1 | |
|---|---|
| p-Toluylendiamin-sulfat | 1,1 |
| 4-Chlorresorcin | 1,0 |
| 4-Amino-3-methylphenol | 0,7 |

(fortgesetzt)

| Phase 1 | |
|---|---|
| m-Aminophenol | 0,2 |
| Texapon® K 1296 PLV | 2,0 |
| Avicel® pH 102 | 30,0 |
| Ammoniumsulfat | 1,0 |
| Arginin | 10,0 |
| D(+)-Lactose | ad 100 |
| Optigel® SH | 15,0 |
| Amaze® | 5,0 |
| Jaguar® HP 120 | 15,0 |
| Luviskol® K30 | 5,00 |

Analog zur Herstellvorschrift des Beispiel 1 wurde eine Tablette gepreßt und mit dem resultierende Färbegel entsprechende Strähnen gefärbt. Die Strähnen wiesen eine mittelblonde Färbung auf.

**Beispiel 4**

| Phase 1 | |
|---|---|
| p-Toluylendiamin-sulfat | 1,6 |
| 1-(β-Hydroxyethyl)-amino-4-methyl-2-nitrobenzol | 0,7 |
| 4-Chlorresorcin | 0,5 |
| 4-Amino-3-methylphenol | 5,4 |
| 2,7-Dihydroxynaphthalin | 1,1 |
| 5-Amino-2-methylphenol | 5,1 |
| Texapon® K 1296 PLV | 2,0 |
| Avicel® pH 102 | 30,0 |
| Ammoniumsulfat | 1,0 |
| Arginin | 10,0 |
| D(+)-Lactose | ad 100 |
| Optigel® SH | 15,0 |
| Amaze® | 5,0 |
| Jaguar® HP 120 | 15,0 |
| Luviskol® K30 | 5,0 |

Analog zur Herstellvorschrift des Beispiel 1 wurde eine Tablette gepreßt und mit dem resultierende Färbegel entsprechende Strähnen gefärbt. Die Strähnen wiesen eine intensive rote Färbung auf.

**Patentansprüche**

1. Formkörper zur Färbung keratinischer Fasern, enthaltend in einem kosmetisch akzeptablen Träger mindestens ein Farbstoffvorprodukt und ein Oxidationsmittel, **dadurch gekennzeichnet, dass** es zusätzlich mindestens ein Alkalisierungsmittel enthält, das eine Aminosäure oder ein Oligopeptid mit mindestens einer Aminogruppe und einer Carboxy- oder eine Sulfogruppe ist, deren 2,5%ige wässrige Lösung einen pH-Wert von größer als 9,0 aufweist.

2. Formkörper nach Anspruch 1, **dadurch gekennzeichnet, dass** das feste Alkalisierungsmittel eine α-Aminosäure ist.

3. Formkörper nach Anspruch 2, **dadurch gekennzeichnet, dass** das Alkalisierungsmittel Arginin ist.

4. Formkörper nach einem der Ansprüche 1 bis 3, **dadurch gekennzeichnet, dass** er als Farbstoffvorprodukt mindestens eine Entwicklerkomponente enthält.

5. Formkörper nach einem der Ansprüche 1 bis 4, **dadurch gekennzeichnet, dass** er als Farbstoffvorprodukt ein Derivat des Indols und/oder des Indolins enthält.

6. Formkörper nach einem der Ansprüche 1 bis 5, **dadurch gekennzeichnet, dass** er als Oxidationsmittel ein Anlagerungsprodukt von Wasserstoffperoxid enthält.

7. Formkörper nach Anspruch 6, **dadurch gekennzeichnet, dass** er als Oxidationsmittel Percarbamid enthält.

8. Formkörper nach einem der Ansprüche 1 bis 7, **dadurch gekennzeichnet, dass** er einen Auflösungsbeschleuniger enthält.

9. Formkörper nach Anspruch 8, **dadurch gekennzeichnet, dass** der Auflösungsbeschleuniger ein Desintegrationshilfsmittel ist.

10. Formkörper nach einem der Ansprüche 1 bis 9, **dadurch gekennzeichnet, dass** er aus mindestens drei Schichten besteht, wobei eine erste Schicht (A) die Farbstoffzubereitung und das Alkalisierungsmittel enthält, eine zweite Schicht (B) eine inerte Trennschicht darstellt und eine dritte Schicht (C) die Oxidationsmittelzubereitung enthält.

11. Formkörper nach Anspruch 10, **dadurch gekennzeichnet, dass** er einen stapelförmigen Schichtaufbau in der Reihenfolge (A), (B), (C) aufweist.

12. Formkörper nach Anspruch 10, **dadurch gekennzeichnet, dass** die Schicht (A) vollständig von der Schicht (B) und diese wiederum vollständig von der Schicht (C) umhüllt.

13. Formkörper nach Anspruch 10, **dadurch gekennzeichnet, dass** die Schicht (C) vollständig von der Schicht (B) und diese wiederum vollständig von der Schicht (A) umhüllt ist.

14. Formkörper nach einem der Ansprüche 1 bis 13, **dadurch gekennzeichnet, dass** einzelne Bestandteile der zu verpressenden Zusammensetzung oder der gesamte Formkörper beschichtet sind.

15. Formkörper nach einem der Ansprüche 1 bis 14, **dadurch gekennzeichnet, dass** sich bei der Auflösung ein Gel bildet.

16. Formkörper nach einem der Ansprüche 1 bis 15, **dadurch gekennzeichnet, dass** er mindestens ein Tensid enthält.

17. Formkörper nach einem der Ansprüche 1 bis 16, **dadurch gekennzeichnet, dass** er von einer Primärverpackung umhüllt ist.

18. Verwendung eines Formkörpers nach einem der Ansprüche 1 bis 16 zur Herstellung eines Mittels zur Färbung keratinischer Fasern.

19. Verfahren zur Färbung keratinischer Fasern, **dadurch gekennzeichnet, dass** ein oder mehrere Formkörper nach einem der Ansprüche 1 bis 16 in Wasser gelöst werden, die resultierende Zubereitung anschließend auf die Fasern aufgetragen und nach einer Einwirkzeit wieder abgespült wird.

## Claims

1. A shaped body for coloring keratin fibers, comprising, in a cosmetically acceptable carrier, at least one dye precursor and an oxidizing agent, **characterized in that** it additionally comprises at least one alkalinizing agent which is an amino acid or an oligopeptide having at least one amino group and a carboxyl or a sulfo group whose 2.5% strength aqueous solution has a pH of greater than 9.0.

2. The shaped body as claimed in claim 1, **characterized in that** the solid alkalinizing agent is an $\alpha$-amino acid.

3. The shaped body as claimed in claim 2, **characterized in that** the alkalinizing agent is arginine.

4. The shaped body as claimed in any of claims 1 to 3, **characterized in that** it comprises at least one developer component as dye precursor.

5. The shaped body as claimed in any of claims 1 to 4, **characterized in that** it comprises a derivative of indole and/or of indoline as dye precursor.

6. The shaped body as claimed in any of claims 1 to 5, **characterized in that** it comprises an addition product of hydrogen peroxide as oxidizing agent.

7. The shaped body as claimed in claim 6, **characterized in that** it comprises percarbamide as oxidizing agent.

8. The shaped body as claimed in any of claims 1 to 7, **characterized in that** it comprises a dissolution accelerator.

9. The shaped body as claimed in claim 8, **characterized in that** the dissolution accelerator is a disintegration auxiliary.

10. The shaped body as claimed in any of claims 1 to 9, **characterized in that** it consists of at least three layers, where a first layer (A) comprises the dye preparation and the alkalinizing agent, a second layer (B) represents an inert interlayer and a third layer (C) comprises the oxidizing agent preparation.

11. The shaped body as claimed in claim 10, **characterized in that** it has a stack-like layer structure in the order (A), (B), (C).

12. The shaped body as claimed in claim 10, **characterized in that** the layer (A) is completely covered by the layer (B), which in turn is completely covered by the layer (C).

13. The shaped body as claimed in claim 10, **characterized in that** the layer (C) is completely covered by the layer (B), which in turn is completely covered by the layer (A).

14. The shaped body as claimed in any of claims 1 to 13, **characterized in that** individual constituents of the composition to be compressed or the overall shaped body are coated.

15. The shaped body as claimed in any of claims 1 to 14, **characterized in that** a gel forms upon dissolution.

16. The shaped body as claimed in any of claims 1 to 15, **characterized in that** it comprises a surfactant.

17. The shaped body as claimed in any of claims 1 to 16, **characterized in that** it is covered by a primary packaging.

18. The use of a shaped body as claimed in any of claims 1 to 16 for the preparation of a composition for coloring keratin fibers.

19. A method of coloring keratin fibers, **characterized in that** one or more shaped bodies as claimed in any of claims 1 to 16 are dissolved in water, the resulting preparation is then applied to the fibers and is rinsed off again after a contact time.

## Revendications

1. Corps façonné pour teindre des fibres kératiniques, contenant dans un véhicule acceptable sur le plan cosmétique, au moins un produit précurseur de colorant et un agent d'oxydation, **caractérisé en ce qu'**il contient en outre au moins un agent d'alcalinisation, qui est un acide aminé ou un oligopeptide ayant au moins un groupe amino et un groupe carboxy ou un groupe sulfo, dont la solution aqueuse à 2,5% présente une valeur de pH supérieure à 9,0.

2. Corps façonné selon la revendication 1, **caractérisé en ce que** l'agent d'alcalinisation solide est un acide $\alpha$-aminé.

3. Corps façonné selon la revendication 2, **caractérisé en ce que** l'agent d'alcalinisation est l'arginine.

4. Corps façonné selon l'une quelconque des revendications 1 à 3, **caractérisé en ce qu'**il contient au moins un composant révélateur en tant que produit précurseur de colorant.

5. Corps façonné selon l'une quelconque des revendications 1 à 4, **caractérisé en ce qu'**il contient un dérivé de l'indole et/ou de l'indoline en tant que produit précurseur de colorant.

6. Corps façonné selon l'une quelconque des revendications 1 à 5, **caractérisé en ce qu'**il contient un produit d'addition du peroxyde d'hydrogène en tant qu'agent d'oxydation.

7. Corps façonné selon la revendication 6, **caractérisé en ce qu'**il contient du percarbamide en tant qu'agent d'oxydation.

8. Corps façonné selon l'une quelconque des revendications 1 à 7, **caractérisé en ce qu'**il contient un accélérateur de dissolution.

9. Corps façonné selon la revendication 8, **caractérisé en ce que** l'accélérateur de dissolution est un adjuvant de délitement.

10. Corps façonné selon l'une quelconque des revendications 1 à 9, **caractérisé en ce qu'**il est constitué d'au moins trois couches, une première couche (A) contenant la préparation de colorant et l'agent d'alcalinisation, une deuxième couche (B) représentant une couche de séparation inerte, et une troisième couche (C) contenant la préparation d'agent d'oxydation.

11. Corps façonné selon la revendication 10, **caractérisé en qu'**il présente au moins une structure stratifiée sous forme de pile dans l'ordre (A), (B), (C).

12. Corps façonné selon la revendication 10, **caractérisé en ce que** la couche (A) est entièrement enveloppée de la couche (B) et celle-ci est de nouveau entièrement enveloppée de la couche (C).

13. Corps façonné selon la revendication 10, **caractérisé en ce que** la couche (C) est entièrement enveloppée de la couche (B) et celle-ci est de nouveau entièrement enveloppée de la couche (A).

14. Corps façonné selon l'une quelconque des revendications 1 à 13, **caractérisé en ce que** différents constituants de la composition à comprimer ou le corps façonné entier sont revêtus.

15. Corps façonné selon l'une quelconque des revendications 1 à 14, **caractérisé en ce qu'**un gel se forme lors de la dissolution.

16. Corps façonné selon l'une quelconque des revendications 1 à 15, **caractérisé en ce qu'**il contient au moins un tensio-actif.

17. Corps façonné selon l'une quelconque des revendications 1 à 16, **caractérisé en ce qu'**il est enveloppé d'un conditionnement primaire.

18. Utilisation d'un corps façonné selon l'une quelconque des revendications 1 à 16 pour préparer un agent pour teindre des fibres kératiniques.

19. Procédé pour teindre des fibres kératiniques, **caractérisé en ce qu'**un ou plusieurs corps façonnés selon l'une quelconque des revendications 1 à 16 sont dissous dans de l'eau, la préparation résultante est appliquée ensuite sur les fibres et après une durée d'action, elle est de nouveau éliminée par rinçage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1530229 B
- JP 46004280 B
- DE OS2215303 A
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- DE 3725030 A
- DE 3723354 A
- DE 3926344 A


### In der Beschreibung aufgeführte Nicht-Patentliteratur


- **MONOGRAPHIE CH. ZVIAK.** The Science of Hair Care. 248-250
- THE SCIENCE OF HAIR CARE. 264-267
- Dermatology. Verlag Marcel Dekker Inc, 1986, vol. 7
- Zerfallsbeschleunigern werden gemäß Römpp. vol. 6, 4440
- **VOIGT.** Lehrbuch der pharmazeutischen Technologie. 1987, 182-184
- **KH. SCHRADER.** Grundlagen und Rezepturen der Kosmetika. Hüthig Buch Verlag, 1989
- Die Kunststoffe und ihre Eigenschaften. Hans Domininghaus. VDI Verlag, 1988, 193